# EUROPEAN PATENT APPLICATION

(11) **EP 3 167 934 A1**
(43) Date of publication of application: **17.05.2017**
(21) Application number: 15819694.9
(22) Date of filing: 10.02.2015
(51) Int. Cl.: A61N 7/00, A61K 49/22

(54) **COUPLING LIQUID SUPPLY EQUIPMENT FOR HIGH-INTENSITY FOCUSED ULTRASOUND THERAPY SYSTEM**

(30) Priority: 08.07.2014 CN 201410323927
(71) Applicant: Shenzhen Pro-hifu Medical Tech. Co., Ltd., Shenzhen, Guangdong 518000 (CN)
(72) Inventor: JIANG, Jianhui, Shenzhen Guangdong 518000 (CN); NIU, Mu, Shenzhen Guangdong 518000 (CN); YANG, Xingwen, Shenzhen Guangdong 518000 (CN)
(74) Representative: Krauns, Christian
(86) International application number: PCT/CN2015/072707
(87) International publication number: WO 2016/004764

(57) **Abstract**

Disclosed is coupling liquid supply equipment for a high-intensity focused ultrasound therapy system, which is applied to the technical field of medical devices and aims to solve the problem in the prior art that it is difficult to obtain an ideal transmission medium during the process of degassed water preparation. The coupling liquid supply equipment comprises a coupling liquid preparation device (1) for preparing a mixing liquid formed by medical alcohol and polyvinylpyrrolidone, a water cycle degassing device (2) communicated with the coupling liquid preparation device (1) and employing a vacuumizing process to prepare degassed water, and a conveying device (3) communicated with the water cycle degassing device (2) and used for conveying the coupling liquid, formed by mixing the mixing liquid and the degassed water, to a high-intensity focused ultrasound therapeutic head (4). The fully-stirred mixing liquid of medical alcohol and polyvinylpyrrolidone is added during the process of degassed water preparation, and the mixing liquid and the degassed water are fully mixed together to guarantee that the coupling liquid conveyed to the high-intensity focused ultrasound therapeutic head (4) has a good transmission medium characteristic.

## Description

### TECHNICAL FIELD

The present invention relates to the technical field of medical instruments, and in particular, relates to a coupling liquid supply apparatus for a high-intensity focused ultrasound therapy system.

### BACKGROUND

At present, the high-intensity focused ultrasound therapy is being more and more extensively applied. The high-intensity focused ultrasound therapeutic technique refers to a therapeutic technique by using a high-intensity focused ultrasound (HIFU) therapy system. The high-intensity focused ultrasound therapy system comprises a power source, a therapy control part a positioning part, and a real-time evaluation part, a motion control portion and the like. The therapeutic theory is mainly to use the heat effect generated by the high-intensity ultrasound focused on the biologic tissues, such that tissues in the focused region for therapy are instantly condensed and die. Instead, the tissues out of the region are subjected to no damage, and the condensed and dead tissues may be finally gradually absorbed or scarred. Such technique for local treatment of the tumors is also referred to as "cutting", and is mainly applicable to treatments of malicious and benign tumors of tissues organs.

Since the ultrasound may only be propagated in a medium, and in the case of a medium cutting face with two different acoustic impedances, reflection and refraction may occur. A greater difference of the acoustic impedances signifies greater reflection and refraction factors. The acoustic impendence of human body tissues is close to the water, alcohol and the like, but is greatly different from the air. Therefore, generally purified degassed water or dedicatedly prepared industrial solution is used as the propagation medium. However, during the preparation of the degassed water, it is very difficult to remove all the gases. When the degassed water is placed still for a period of time, the gases would be slowly dissolved into the water again. In addition, during the high-intensity focused ultrasound therapy, when the high-intensity ultrasound passes through the degassed water, a cavitation effect may be caused, and after a large number of micro gas bubbles are generated, gas bubbles which affect the ultrasound propagation may be generated via fusion of the micro gas bubbles. Further, the dedicatedly prepared industrial solution is troublesome in preparation and has a high cost. In addition, the industrial solution is generally simply volatile, simply combustible and toxic and is hard to be stored. Generally, the industrial solution needs to be used in a sealed vacuum environment, which thus imposes higher requirements on the mechanism of the therapy system, and is also subjected to some safety latent risks.

### SUMMARY

The present invention is intended to provide a coupling fluid supply apparatus for a high-intensity focused ultrasound therapy system, wherein a mixed fluid formed by medical alcohol and polyvinylpyrrolidone is added during the preparation of degassed water, such that the mixed fluid are sufficiently fused with the degassed water. The technical solution of the present invention is directed to solving the problem that an ideal propagation medium is hard to be obtained during the preparation of the degassed water in the prior art.

The present invention is implemented by a coupling fluid supply apparatus for a high-intensity focused ultrasound therapy system, comprising: a coupling fluid preparation device configured to prepare a mixed fluid formed by medical alcohol and polyvinylpyrrolidone, a water circulation degassing device communicated with the coupling fluid preparation device and employing a vacuumizing process to prepare degassed water, and a conveying device communicated with the water circulation degassing device and configured to convey the coupling fluid formed by fusing the mixed fluid with the degassed water to a high-intensity focused ultrasound therapeutic head; wherein the water circulation degassing device comprises a water tank communicated with the coupling fluid preparation device and the conveying device to form the coupling fluid, a water-gas separator communicated with the water tank via a water pipe and forming a circulation channel with the water tank, a water circulation vacuum pump communicated with a gas intake channel and a water intake channel of the circulation channel and disposed on the circulation channel and positioned between the gas intake channel and the water-gas separator; and the coupling fluid preparation device comprises a fluid reservoir communicated with the water tank via the water pipe, the fluid reservoir being provided with an automatic agitator and provided with a raw material feeding port for adding the mixed fluid.

Further, the coupling fluid supply device further comprises a solenoid valve disposed on the water pipe to communicate an outlet of the fluid reservoir with an inlet of the water tank.

Further, the water circulation degassing device further comprises a water level gauge disposed on the water tank to measure a water level in the water tank, and a vacuum gauge disposed on the water tank to measure a vacuum degree in the water tank.

Further, the coupling fluid supply apparatus for a high-intensity focused ultrasound therapy system further comprises a first control valve and a first adapter; wherein a gas exhaust of the water-gas separator is communicated with a gas intake of the water tank, via a pipeline through the water circulation vacuum pump, the first control valve and the first adapter, to form a gas removing channel, the water intake channel is mounted at the first adapter and communicated with the gas removing channel, and the first control valve is disposed between the first adapter and the water circulation vacuum pump.

Further, the coupling fluid supply apparatus for a high-intensity focused ultrasound therapy system further comprises a magnetic pump, a second adapter, a second control valve and a third adapter; wherein a first water outtake of the water tank is communicated with a water inlet of the water-gas separator, via the pipeline through the magnetic pump, the second adapter, the second control valve and the third adapter, to form a water exhaust channel, the water exhaust channel forming the circulation channel with the gas removing channel.

Further, the coupling fluid supply apparatus for a high-intensity focused ultrasound therapy system further comprises a shutoff valve and a third control valve; wherein the pipeline is communicated with the shutoff valve, the third control valve, the first adapter, the first control valve, the water circulation vacuum pump and the gas intake of the water-gas separator to form the gas intake channel.

Further, the coupling fluid supply apparatus for a high-intensity focused ultrasound therapy system further comprises a water intake ball valve, a fourth control valve and a filter; wherein the pipeline is communicated with the water intake valve, the fourth control valve, the filter, the third adapter, the second control valve, the second adapter and the water intake of the water tank to form the water intake channel.

Further, the conveying device comprises a fluid conveying channel configured to conveying the coupling fluid to the high-intensity focused ultrasound therapeutic head, a sewage exhaust channel communicated with the fluid conveying channel and having a sewage exhaust, a fifth control valve and a peristaltic pump; wherein a second water outtake of the water tank is communicated with a water bag of the high-intensity focused ultrasound therapeutic head, via a pipeline through the fifth control valve and the peristaltic pump, to form the liquid conveying channel.

Further, the conveying device further comprises a fourth adapter, a sixth control valve and a self-priming pump communicating the sewage exhaust channel with the fluid conveying channel; and the sewage exhaust channel comprises a first sewage exhaust channel formed with the sewage exhaust via the pipeline through the fourth adapter, the sixth control valve and the self-priming pump, the fourth adapter being disposed between the fifth control valve and the peristaltic pump.

Further, the conveying device further comprises a fifth adapter and a seventh control valve communicating the sewage exhaust channel with the fluid conveying channel; and the sewage exhaust channel further comprises a second sewage exhaust channel formed with the sewage exhaust via the pipeline through the fifth adapter, the seventh control valve and the self-priming pump, the fifth adapter being disposed between the peristaltic pump and the water bag, a sixth adapter being disposed between the seventh control valve and the self-priming pump, the sixth control valve and the seventh control valve being communicated with the self-priming pump via the sixth adapter.

The present invention achieves the following beneficial effects over the prior art: By using the coupling fluid preparation device having an automatic agitator, the medical alcohol and the polyvinylpyrrolidone are sufficiently mixed, the gas in water is removed by using the water-gas separator in the water circulation degassing device and the coupling fluid is sufficiently fused with the degassed water, and the coupling fluid is conveyed to the high-intensity focused ultrasound therapeutic head by using the conveying device as an ultrasound propagation medium. A mixed fluid of the medical alcohol and the polyvinylpyrrolidone that are sufficiently mixed is added during the preparation of the degassed water, such that the mixed fluid is sufficiently fused with the degassed water. This ensures that the coupling fluid in the high-intensity ultrasound therapeutic head has a good propagation medium characteristic.

### BRIEF DESCRIPTION OF THE DRAWINGS

FIG. 1 is a schematic structural view of a coupling fluid supply device for a high-intensity focused ultrasound therapy system according to an embodiment of the present invention.
FIG. 2 is a schematic structural view of a water circulation degassing device of the coupling fluid supply device in FIG. 1.
FIG. 3 is a schematic structural view of a conveying device of the coupling fluid supply device in FIG. 1.

### DETAILED DESCRIPTION OF EMBODIMENTS

To make the objective, technical solution, and advantages of the present invention clearer, the following section describes the technical solutions of the present invention in combination with the accompanying drawings and embodiments. It should be understood that the embodiments described here are only exemplary ones for illustrating the present invention, and are not intended to limit the present invention.

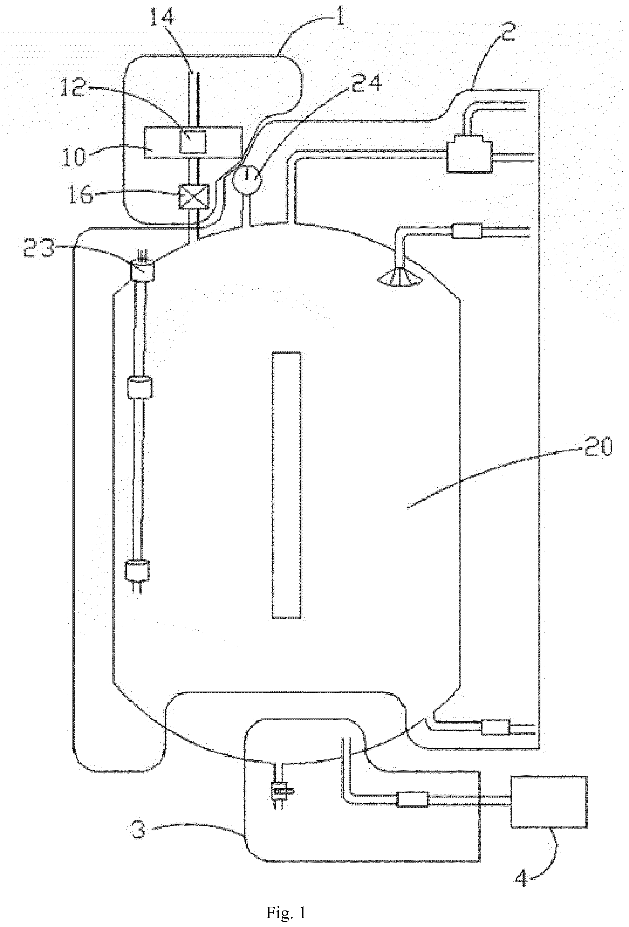

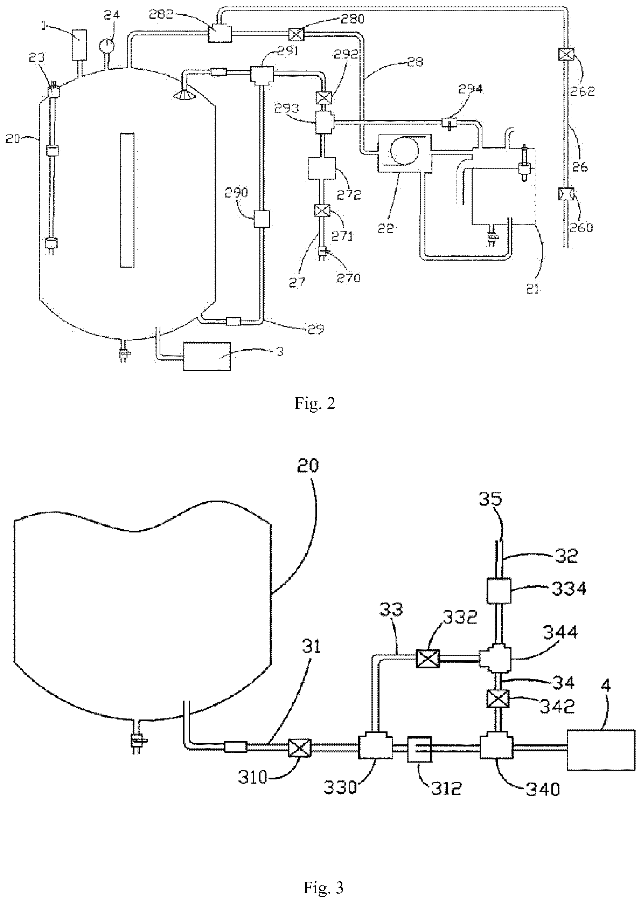

Referring to FIG. 1 and FIG. 2, a coupling fluid supply apparatus for a high-intensity focused ultrasound therapy system according to an embodiment of the present invention comprises: a coupling fluid preparation device 1 configured to prepare a mixed fluid formed by medical alcohol and polyvinylpyrrolidone, a water circulation degassing device 2 communicated with the coupling fluid preparation device 1 and employing a vacuumizing process to prepare degassed water, and a conveying device 3 communicated with the water circulation degassing device 2 and configured to convey the coupling fluid formed by fusing the mixed fluid with the degassed water to a high-intensity focused ultrasound therapeutic head 4; wherein the water circulation degassing device 2 comprises a water tank 20 communicated with the coupling fluid preparation device 1 and the conveying device 3 to form the coupling fluid, a water-gas separator 21 communicated with the water tank 20 via a water pipe and forming a circulation channel 25 with the water tank 20, a water circulation vacuum pump 22 communicated with a gas intake channel 26 and a water intake channel 27 of the circulation channel 25 and disposed on the circulation channel 25 and positioned between the gas intake channel 26 and the water-gas separator 21; and the coupling fluid preparation device 1 comprises a fluid reservoir 10 communicated with the water tank 20 via the water pipe, the fluid reservoir 10 being provided with an automatic agitator 12 and provided with a raw material feeding port 14 for adding the mixed fluid.

During the use, the automatic agitator 12 in the coupling fluid preparation device 1 mixes the medical alcohol and the polyvinylpyrrolidone, and meanwhile turns off the water intake channel 27 to introduce an external water source into the water tank 20; and when the water level in the water tank 20 reaches a predetermined value, the water intake channel 27 is turned off. In this case, the coupling fluid preparation device 1 is turned on, such that the mixed fluid after sufficient mixing flows into the water tank 20 via the water pipe; and afterwards, the water pipe is turned off to stop the mixed fluid from flowing into the water tank 20. The water-gas separator 21 in the water circulation degassing device 2 is started, to remove the gas in the water in one aspect, and cause the coupling fluid to be sufficiently fused with the degassed water in another aspect. The conveying device 3 is started to convey the coupling fluid to the high-intensity focused therapeutic head 4 as an ultrasound propagation medium during the therapy. In this embodiment, the predetermined value may be a maximum water level the water tank 20 can accommodate or any predetermined water level.

In this embodiment, the medical alcohol has a concentration of 95%, and a mass ratio of the medical alcohol to the polyvinylpyrrolidone in the mixed fluid is 5:1. The mixed fluid is injected to the water tank 10 via the raw material feeding port 14, and is mixed via an automatic agitator 12, such that the polyvinylpyrrolidone is uniformly dissolved in the medical alcohol.

The polyvinylpyrrolidone, also referred to as PVP, is a hydrophilic polymer, which is mainly used for the preparation of a base portion of a coupling agent and achieves an effect of a gel. Since the polyvinylpyrrolidone is a macromolecule polymer, the molecular weight thereof is not fixed. During the use, selection is made according to an average molecular weight, and the selection identifier is judged by the letter K plus a digit. The large the digit is, the greater the average molecular weight is, and the thicker the generated gel is. Preferably, the polyvinylpyrrolidone used in the embodiments of the present invention is PVP K29-32 which has a molecular weight of 58 000, and the function thereof is to absorb the gas dissolved in the water and inhibit the cavitation of the water during the ultrasound propagation. The medical alcohol, also referred to as ethanol, is mainly to accelerate dissolving of the polyvinylpyrrolidone, and meanwhile may inhibit the growth of the bacteria and further reduce the cooling point.

In the coupling fluid supply apparatus for a high-intensity focused ultrasound therapy system according to the embodiments of the present invention, the coupling fluid preparation device having the automatic agitator 12 sufficiently mixes the medical alcohol and the polyvinylpyrrolidone; gas in the water is removed by using the water-gas separator 21 in the water circulation degassing device 2 such that the coupling fluid is sufficiently fused with the degassed water; and the coupling fluid is conveyed to the high-intensity focused ultrasound therapeutic head 4 via the conveying device 3 as a ultrasound propagation medium. The mixed fluid of the medical alcohol and the polyvinylpyrrolidone that are sufficiently mixed is added during the preparation of the degassed water, such that the mixed fluid is sufficiently fused with the degassed water. This ensures that the coupling fluid in the high-intensity ultrasound therapeutic head 4 has a good propagation medium characteristic.

Referring to FIG. 1 and FIG. 2, the coupling fluid supply device 1 further comprises a solenoid valve 16 disposed on the water pipe to communicate an outlet of the fluid reservoir 10 with an inlet of the water tank 20. It should be understood that the solenoid valve 16 is employed to turn on or turn off the water pipe, such that a mixed fluid of the medical alcohol and the polyvinylpyrrolidone is injected into the water tank 20 in a timing and quantitative manner. In other embodiments, the solenoid valve 16 may also be replaced with another control switch, as long as the switch is capable of implementing turn-on and turn-off of the injection of the mixed fluid.

Referring to FIG. 1 and FIG. 2, the water circulation degassing device 2 further comprises a water level gauge 23 disposed on the water tank 20 to measure a water level in the water tank 20, and a vacuum gauge 24 disposed on the water tank 20 to measure a vacuum degree in the water tank 20. It should be appreciated that the water level gauge 23 is used to measure the water level in the water tank 20. When an external water source is introduced via the water intake channel, the water level gauge 23 controls a predetermined water level value in the water tank 20, such that the water level in the water tank 20 is directly controlled via display value on the water level gauge 23. The vacuum gauge 24 is disposed on the water tank 20 to measure the vacuum degree in the water tank 20, which prevents the case where a desired vacuum degree fails to be achieved and thus an ideal ultrasound propagation medium characteristic fails to be reached because the vacuum degree in the water tank 20 is too large or too small during the vacuumizing process.

Referring to FIG. 2, the coupling fluid supply apparatus for a high-intensity focused ultrasound therapy system further comprises a first control valve 280 and a first adapter 282; wherein a gas exhaust of the water-gas separator 21 is communicated with a gas intake of the water tank 20, via a pipeline through the water circulation vacuum pump 22, the first control valve 280 and the first adapter 282, to form a gas removing channel 28, the water intake channel 26 is mounted at the first adapter 282 and communicated with the gas removing channel 28, and the first control valve 280 is disposed between the first adapter 282 and the water circulation vacuum pump 22. It should be understood that the first adapter 282 is employed such that the gas intake channel 26 and the gas removing channel 28 share the same segment of pipeline, the first adapter 282 is disposed adjacent to the gas intake of the water tank 20, and the first control valve 280 turns off the gas removing channel 28 when the gas intake channel 26 operates, such that the air flows along the gas intake channel 26 and through the first adapter 282 and the pipeline into the gas intake of the water tank 20, thereby implementing gas charging of the water tank 20; when the gas removing channel 28 operates, the first control valve 280 is in a turn-on state and the gas intake channel 26 is in a turn-off state, the water-gas separator 21 feeds the gas generated during the preparation of the degassed water, via the water circulation vacuum pump 22 and along the pipeline, the first control valve 280 and the first adapter 282, into the water tank 20. In this embodiment, the first control valve 280 may be a flow control valve or a solenoid valve to control turn-on or turn-off of the pipeline; and the first adapter 282 is a three-way adapter, such that the gas removing channel 28 and the gas intake channel 26 do not cause interference to each other during operation.

Referring to FIG. 2, the coupling fluid supply apparatus for a high-intensity focused ultrasound therapy system further comprises a magnetic pump 290, a second adapter 291, a second control valve 292 and a third adapter 293; wherein a first water outtake of the water tank 20 is communicated with a water inlet of the water-gas separator 21, via the pipeline through the magnetic pump 290, the second adapter 291, the second control valve 292 and the third adapter 293, to form a water exhaust channel 29, the water exhaust channel 29 forming the circulation channel 25 with the gas removing channel 28. It may be understood that the second adapter 291 and the third adapter 293 are employed such that the water exhaust channel 29 and the water intake channel 27 share the same segment of pipeline. The circulation channel 25 of the water circulation degassing device 2 is formed by the water exhaust channel 29 and the gas removing channel 28, thereby ensuring circulative progress of the entire process. In this embodiment, the second adapter 291 and the third adapter 293 are both a three-way adapter, such that the water exhaust channel 29 and the water intake channel 27 do not cause interference to each other during operation. When the magnetic pump 290 and the second control valve 292 are turned on, the fluid in the water tank 20 flows into the water-gas separator 21 along the water exhaust channel 29. In this embodiment, a water intake ball valve 294 is further disposed between the third adapter 293 and the water-gas separator 21, while the magnetic pump 290 and the second control valve 292 are turned on, the water intake ball valve 294 is turned on, such that the fluid in the water tank 20 flows into the water-gas separator 21 via the water exhaust channel 29. The water intake ball valve 294 is arranged herein to prevent the fluid from flowing into the water intake channel 27.

Referring to FIG. 2, the coupling fluid supply apparatus for a high-intensity focused ultrasound therapy system further comprises a shutoff valve 260 and a third control valve 262; wherein the pipeline is communicated with the shutoff valve 260, the third control valve 262, the first adapter 282, the first control valve 280, the water circulation vacuum pump 22 and the gas intake of the water-gas separator 21 to form the gas intake channel 26. It may be understood that the third control valve 262 is turned off to turn off the gas intake channel 26, such that the external gas flows into the water circulation degassing device 2 along the gas intake channel 26. The third control valve 262 is a flow control valve or a solenoid valve to control turn-on or turn-off of the pipeline. The shutoff valve 260 is employed to control the flow of the external gas flowing into the gas intake channel 26.

Referring to FIG. 2, the coupling fluid supply apparatus for a high-intensity focused ultrasound therapy system further comprises a water intake ball valve 270, a fourth control valve 271 and a filter 272; wherein the pipeline is communicated with the water intake valve 270, the fourth control valve 271, the filter 272, the third adapter 293, the second control valve 292, the second adapter 291 and the water intake of the water tank 20 to form the water intake channel 27. It may be understood that the water intake channel 27 is employed to introduce an external water source into the water tank 20; and the water intake ball valve 270, the fourth control valve 271 and the filter 272 are sequentially disposed in the water intake channel 27, such that the external water source flows into the water tank 20 through the second control valve 292 and the second adapter 291. When the water intake ball valve 270 is turned on, the magnetic pump 290 and the water intake ball valve in the water exhaust channel 29 are in a turn-off state, and the external water source flows into the water tank 20 along the water intake channel 27. The water intake channel 27 is turned on, such that the water level value in the water tank 20 reaches a predetermined value.

Referring to FIG. 1 and FIG. 3, the conveying device 3 comprises a fluid conveying channel 31 configured to conveying the coupling fluid to the high-intensity focused ultrasound therapeutic head 4, a sewage exhaust channel 32 communicated with the fluid conveying channel 31 and having a sewage exhaust 35, a fifth control valve 310 and a peristaltic pump 312; wherein a second water outtake of the water tank 20 is communicated with a water bag (not illustrated) of the high-intensity focused ultrasound therapeutic head 4, via a pipeline through the fifth control valve 310 and the peristaltic pump 312, to form the liquid conveying channel 31. It may be understood that the fluid conveying channel 31 is communicated with the sewage exhaust channel 32, such that the extra coupling fluid is removed from the fluid conveying channel 31 via the sewage exhaust channel 32. This ensures that the coupling fluid coming from the water tank 20 each time has an optimal propagation medium characteristic. The fifth control valve 310 and the peristaltic pump 312 are disposed on the fluid conveying channel 31; and the fifth control valve 310 and the peristaltic pump 312 are turned on, such that the coupling fluid is conveyed to the water bag of the high-intensity focused ultrasound therapeutic head 4.

Referring to FIG. 1 and FIG. 3, the conveying device 3 further comprises a fourth adapter 330, a sixth control valve 332 and a self-priming pump 334 communicating the sewage exhaust channel 32 with the fluid conveying channel 31; and the sewage exhaust channel 32 comprises a first sewage exhaust channel 33 formed with the sewage exhaust 35 via the pipeline through the fourth adapter 330, the sixth control valve 332 and the self-priming pump 334, the fourth adapter 330 being disposed between the fifth control valve 310 and the peristaltic pump 312. It may be understood that the first sewage exhaust channel 33 is formed via the pipeline through the first adapter 330, the sixth control valve 332 and the self-priming pump 334; the fourth adapter 330 is disposed between the fifth control valve 310 and the peristaltic pump 312, and the remaining coupling fluid in the fifth control valve 310 is removed via the first sewage exhaust channel 33.

Referring to FIG. 1 and FIG. 3, the conveying device 3 further comprises a fifth adapter 340 and a seventh control valve 342 communicating the sewage exhaust channel 32 with the fluid conveying channel 31; and the sewage exhaust channel 32 further comprises a second sewage exhaust channel 34 formed with the sewage exhaust 35 via the pipeline through the fifth adapter 340, the seventh control valve 342 and the self-priming pump 334, the fifth adapter 340 being disposed between the peristaltic pump 312 and the water bag, a sixth adapter 344 being disposed between the seventh control valve 342 and the self-priming pump 334, the sixth control valve 332 and the seventh control valve 342 being communicated with the self-priming pump 334 via the sixth adapter 344. It should be understood that the second sewage exhaust channel 34 is formed via the pipeline through the fifth adapter 340, the seventh control valve 342 and the self-priming pump 334; the fifth adapter 340 is employed such that the second sewage exhaust channel 34 is communicated with the fluid conveying channel 31, and the remaining coupling fluid in the peristaltic pump 312 is removed via the second sewage exhaust channel 34. The sixth adapter 344 is employed such that the first sewage exhaust channel 33 and the second sewage exhaust channel 34 share the self-priming pump 334, and the sixth control valve 332 and the seventh control valve 342 may be simultaneously or sequentially turned on, to simultaneously or sequentially remove the remaining coupling fluids in the fifth control valve 310 and the peristaltic pump 312. By disposing the second sewage exhaust channel 34, the remaining coupling fluid in the fifth control valve 310 may also be further removed.

Described above are merely preferred embodiments of the present invention, but are not intended to limit the present invention. Any modification, equivalent replacement, or improvement made without departing from the spirit and principle of the present invention should fall within the protection scope of the present invention.

## Claims

1. A coupling fluid supply apparatus for a high-intensity focused ultrasound therapy system, **characterized by** comprising: a coupling fluid preparation device configured to prepare a mixed fluid formed by medical alcohol and polyvinylpyrrolidone, a water circulation degassing device communicated with the coupling fluid preparation device and employing a vacuumizing process to prepare degassed water, and a conveying device communicated with the water circulation degassing device and configured to convey the coupling fluid formed by fusing the mixed fluid with the degassed water to a high-intensity focused ultrasound therapeutic head; wherein the water circulation degassing device comprises a water tank communicated with the coupling fluid preparation device and the conveying device to form the coupling fluid, a water-gas separator communicated with the water tank via a water pipe and forming a circulation channel with the water tank, a water circulation vacuum pump communicated with a gas intake channel and a water intake channel of the circulation channel and disposed on the circulation channel and positioned between the gas intake channel and the water-gas separator; and the coupling fluid preparation device comprises a fluid reservoir communicated with the water tank via the water pipe, the fluid reservoir being provided with an automatic agitator and provided with a raw material feeding port for adding the mixed fluid.

2. The coupling fluid supply apparatus for a high-intensity focused ultrasound therapy system according to claim 1, **characterized in that** the coupling fluid supply device further comprises a solenoid valve disposed on the water pipe to communicate an outlet of the fluid reservoir with an inlet of the water tank.

3. The coupling fluid supply apparatus for a high-intensity focused ultrasound therapy system according to claim 1, **characterized in that** the water circulation degassing device further comprises a water level gauge disposed on the water tank to measure a water level in the water tank, and a vacuum gauge disposed on the water tank to measure a vacuum degree in the water tank.

4. The coupling fluid supply apparatus for a high-intensity focused ultrasound therapy system according to claim 1, **characterized by** further comprising a first control valve and a first adapter; wherein a gas exhaust of the water-gas separator is communicated with a gas intake of the water tank, via a pipeline through the water circulation vacuum pump, the first control valve and the first adapter, to form a gas removing channel, the water intake channel is mounted at the first adapter and communicated with the gas removing channel, and the first control valve is disposed between the first adapter and the water circulation vacuum pump.

5. The coupling fluid supply apparatus for a high-intensity focused ultrasound therapy system according to claim 4, **characterized by** further comprising a magnetic pump, a second adapter, a second control valve and a third adapter; wherein a first water outtake of the water tank is communicated with a water inlet of the water-gas separator, via the pipeline through the magnetic pump, the second adapter, the second control valve and the third adapter, to form a water exhaust channel, the water exhaust channel forming the circulation channel with the gas removing channel.

6. The coupling fluid supply apparatus for a high-intensity focused ultrasound therapy system according to claim 4, **characterized by** further comprising a shutoff valve and a third control valve; wherein the pipeline is communicated with the shutoff valve, the third control valve, the first adapter, the first control valve, the water circulation vacuum pump and the gas intake of the water-gas separator to form the gas intake channel.

7. The coupling fluid supply apparatus for a high-intensity focused ultrasound therapy system according to claim 4, **characterized by** further comprising a water intake ball valve, a fourth control valve and a filter; wherein the pipeline is communicated with the water intake valve, the fourth control valve, the filter, the third adapter, the second control valve, the second adapter and the water intake of the water tank to form the water intake channel.

8. The coupling fluid supply apparatus for a high-intensity focused ultrasound therapy system according to claim 1, **characterized in that** the conveying device comprises a fluid conveying channel configured to conveying the coupling fluid to the high-intensity focused ultrasound therapeutic head, a sewage exhaust channel communicated with the fluid conveying channel and having a sewage exhaust, a fifth control valve and a peristaltic pump; wherein a second water outtake of the water tank is communicated with a water bag of the high-intensity focused ultrasound therapeutic head, via a pipeline through the fifth control valve and the peristaltic pump, to form the liquid conveying channel.

9. The coupling fluid supply apparatus for a high-intensity focused ultrasound therapy system according to claim 8, **characterized in that** the conveying device further comprises a fourth adapter, a sixth control valve and a self-priming pump communicating the sewage exhaust channel with the fluid conveying channel; and the sewage exhaust channel comprises a first sewage exhaust channel formed with the sewage exhaust via the pipeline through the fourth adapter, the sixth control valve and the self-priming pump, the fourth adapter being disposed between the fifth control valve and the peristaltic pump.

10. The coupling fluid supply apparatus for a high-intensity focused ultrasound therapy system according to claim 9, **characterized in that** the conveying device further comprises a fifth adapter and a seventh control valve communicating the sewage exhaust channel with the fluid conveying channel; and the sewage exhaust channel further comprises a second sewage exhaust channel formed with the sewage exhaust via the pipeline through the fifth adapter, the seventh control valve and the self-priming pump, the fifth adapter being disposed between the peristaltic pump and the water bag, a sixth adapter being disposed between the seventh control valve and the self-priming pump, the sixth control valve and the seventh control valve being communicated with the self-priming pump via the sixth adapter.
